# FASCICULE DE BREVET EUROPEEN

(11) **EP 1 618 868 B1**
(45) Date de publication et mention de la délivrance du brevet: **04.11.2015**
(21) Numéro de dépôt: 05291122.9
(22) Date de dépôt: 25.05.2005
(51) Int. Cl.: A61K 8/35, A61K 8/37, A61K 8/49, A61Q 17/04

(54) **Procédé de photostabilisation d'un dérivé de dibenzoylméthane par un dérivé arylalkyl benzoate et un composé bis-résorcinyl triazine et compositions cosmétiques photoprotectrices**
Verfahren zur Photostabilisierung von einen Dibenzoylmethanderivat mit einem Arylalkyl-benzoat-Derivat und einer Bisresorcinyltriazin-Verbindung und kosmetische Sonnenschutzzusammensetzungen
Process for photostabilising a dibenzoylmethane derivative with an arylalkyl benzoate derivative and a bis-resorcinyl triazine compound and sunscreening cosmetic compositions

(30) Priorité: 02.07.2004 FR 0451419
(43) Date de publication de la demande: 25.01.2006
(73) Titulaire: L'Oréal, 75008 Paris (FR)
(72) Inventeur: Candau, Didier, 91570 Bievres (FR)
(74) Mandataire: Miszputen, Laurent

(56) Documents cités:
- WO-A-02/17873
- WO-A-03/039510
- WO-A-2005/009341
- WO-A-2005/117824
- FR-A- 2 800 991
- ISP: "x-tend 226"[Online] août 2003 (2003-08), XP002320817 Extrait de l'Internet: URL:http://www.ispcorp.com> [extrait le 2005-03-10]
- CHATELAIN E ET AL: "PHOTOSTABILIZATION OF BUTYL METHOXYDIBENZOYLMETHANE (AVOBENZONE) AND ETHYLHEXYL METHOXYCINNAMATE BY BIS-ETHYLHEXYLOXYPHENOL METHOXYPHENYL TRIAZINE (TINOSORB S), A NEW UV BROADBAND FILTER" PHOTOCHEMISTRY AND PHOTOBIOLOGY, OXFORD, GB, vol. 74, no. 3, septembre 2001 (2001-09), pages 401-406, XP001057313 ISSN: 0031-8655

## Description

La présente invention est relative à un procédé de photostabilisation du 4-(tert-butyl)-4'-méthoxydibenzoylméthane vis-à-vis du rayonnement UV par le 2-phenylethyl benzoate et le composé bis-résorcinyl triazine 2,4-bis {[4-(2-éthyl-hexyloxy)-2-hydroxy]-phényl}-6-(4-méthoxy-phényl)-1,3,5-triazine.

Elle concerne également de nouvelles compositions, en particulier des compositions cosmétiques à usage topique.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B. La majorité de ces filtres est liposoluble.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de « PARSOL 1789 » par la Société ROCHE VITAMINS. Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

Les dérivés du dibenzoylméthane sont des filtres solides solubles dans les huiles. Parmi les huiles capables de solubiliser efficacement ces filtres UV on connaît les benzoates d'alcools notamment les C12/C15 alkyl benzoates comme les produits commerciaux FINSOLV TN ou WITCONOL APM fabriqués et vendus par la société WITCO.

Cependant, les benzoates d'alcools connus jusqu'à présent ne permettent pas de résoudre le problème de la photostabilité des dérivés du dibenzoylméthane vis-à-vis du rayonnement UV.

On connaît dans la demande WO2005/117824 des émulsions huile/eau comprenant l'association d'au moins un filtre triazine au 2-phenylethylbenzoate et notamment deux exemples 7 et 19 particuliers comprenant l'association 2-phenylethyl Benzoate, Bis-Ethylhexyloxyphenol Methoxyphenyl Triazine et Butylmethoy Dibenzoylmethane.

Or, la Demanderesse vient maintenant de découvrir, de façon surprenante, qu'en associant aux dérivés du dibenzoylméthane mentionnés ci-dessus une quantité efficace d'un composé arylalkyl benzoate et d'un composé bis-résorcinyl triazine, il était possible d'améliorer de manière substantielle et remarquable, la stabilité photochimique (ou photostabilité) de ces mêmes dérivés du dibenzoylméthane. Cette découverte, essentielle, est à la base de la présente invention.

Ainsi, conformément à l'un des objets de la présente invention, il est maintenant proposé un procédé pour améliorer la stabilité du 4-(tert.-butyl)-4'-méthoxydibenzoylméthane vis-à-vis du rayonnement UV consistant à associer audit dibenzoylméthane au moins un composé arylalkyl benzoate et d'au moins un composé bis-résorcinyl triazine.

Un autre objet de l'invention concerne également une composition cosmétique ou dermatologique, à usage topique, caractérisée par le fait qu'elle comprend au moins, dans un support cosmétiquement acceptable :
(a) le 4-(tert-butyl)-4'-méthoxydibenzoylméthane et
(b) le 2-phenylethyl benzoate et;
(c) composé bis-résorcinyl triazine composé bis-résorcinyl triazine 2,4-bis {[4-(2-éthyl-hexyloxy)-2-hydroxy]-phényl}-6-(4-méthoxy-phényl)-1,3,5-triazine , à l'exclusion des compositions particulières mentionnées dans la revendication 9.

D'autres caractéristiques, aspects et avantages de l'invention apparaîtront à la lecture de la description détaillée qui va suivre.

Selon l'invention, on utilise le 4-(ter.-butyl) 4'-méthoxy dibenzoylméthane ou Butyl Methoxy Dibenzoylmethane, proposé à la vente sous la dénomination commerciale de "PARSOL 1789" par la Société Roche Vitamins ; ce filtre répond à la formule suivante :

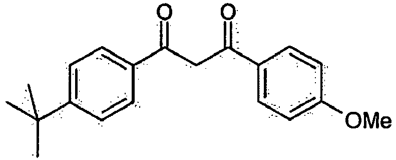

Le composé dibenzoylméthane peut être présents dans les compositions conformes à l'invention à des teneurs qui varient de préférence de 0,01 à 10% en poids et plus préférentiellement de 0,1 à 6% en poids par rapport au poids total de la composition.

Les composés dérivés arylalkyl benzoates comme le 2-phenylethyl benzoate conformes à l'invention et leurs synthèses sont connus depuis longtemps dans la littérature chimique et notamment dans le brevet PL55230.

Selon l'invention, on utilise le benzoate de 2-éthylphenyle 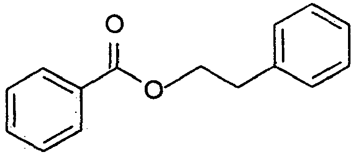 comme le produit commercial X-TEND 226 ® vendu par la société ISP.

Le 2-phenylethyl benzoate conformes à l'invention peut être présent dans les compositions conformes à l'invention à des teneurs allant de 0,1 à 40% en poids et plus préférentiellement de 0,1 à 30 % en poids par rapport au poids total de la composition.

Selon l'invention, on utilise le composé 2,4-bis {[4-(2-éthyl-hexyloxy)-2-hydroxy]-phényl)-6-(4-méthoxy-phényl)-1,3,5-triazine ou Bis-Ethylhexyloxyphenol Methoxyphenyl Triazine (nom INCI) tel que le produit vendu sous le nom commercial «TINOSORB S » par CIBA GEIGY,

Le composés bis-résorcinyl triazine est généralement présent dans les compositions filtrantes selon l'invention à une concentration allant de de 0,1 à 20% en poids et plus préférentiellement de 1 à 10% en poids et plus particulièrement de 2 à 8% en poids par rapport au poids total de la composition.

Selon la présente invention, le mélange photostabilisant composé arylalkyl benzoate/ bis-résorcinyl triazine sera utilisé dans une quantité suffisante permettant d'obtenir une amélioration notable et significative de la photostabilité du dérivé du dibenzoylméthane dans une composition donnée. Cette quantité minimale en agent photostabilisant à mettre en oeuvre peut varier selon la quantité de dibenzoylméthane présent au départ dans la composition et selon la nature du support cosmétiquement acceptable retenu pour la composition. Elle peut être déterminée sans aucune difficulté au moyen d'un test classique de mesure de photostabilité.

Les compositions conformes à l'invention peuvent comporter en plus d'autres agents photoprotecteurs organiques ou inorganiques complémentaires actifs dans l'UVA et/ou l'UVB hydrosolubles ou liposolubles ou bien insolubles dans les solvants cosmétiques couramment utilisés.

Les agents photoprotecteurs organiques complémentaires sont notamment choisis parmi les anthranilates ; les dérivés cinnamiques ; les dérivés salicyliques, les dérivés du camphre; les dérivés de la benzophénone ; les dérivés de β,β-diphénylacrylate ; les dérivés de triazine autres que ceux du type bis-résorcinyl triazine ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP, Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés de β ,β-diphénylacrylate:

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11» par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés de triazine :

- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

### Dérivés anthraniliques :

Menthyl anthranilate vendu sous le nom commercial commercial « NEO HELIOPAN MA » par Haarmann et REIMER,

Dérivés d'imidazolines :
Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les agents photoprotecteurs organiques complémentaires préférentiels sont choisis parmi
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Ethylhexyl triazone,
Diethylhexyl Butamido Triazone,
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les agents photoprotecteurs complémentaires inorganiques sont choisis parmi des pigments et plus préférentiellement encore des nanopigments (taille moyenne des particules primaires : généralement entre 5 nm et 100 nm, de préférence entre 10 nm et 50 nm) d'oxydes métalliques traités ou non comme par exemple des nanopigments d'oxyde de titane (amorphe ou cristallisé sous forme rutile et/ou anatase), de fer, de zinc, de zirconium ou de cérium .

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par:
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tioveil MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212» de la société KEMIRA,
- l'alumine et la silicone tels que le produit « UVT-M262» de la société KEMIRA.
   D'autres nanopigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 CARDRE UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.
   Les nanopigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".
   Les nanopigments d'oxyde de zinc non enrobés, sont par exemple
- ceux commercialisés sous la dénomination "Z-COTE" par la société SUNSMART ;
- ceux commercialisés sous la dénomination "NANOX" par la société ELEMENTIS ;
- ceux commercialisés sous la dénomination "NANOGARD WCD 2025" par la société NANOPHASE TECHNOLOGIES ;

Les nanopigments d'oxyde de zinc enrobés sont par exemple
- ceux commercialisés sous la dénomination "OXIDE ZINC CS-5" par la société Toshibi (ZnO enrobé par polymethylhydrogenesiloxane) ;
- ceux commercialisés sous la dénomination "NANOGARD ZINC OXIDE FN" par la société NANOPHASE TECHNOLOGIES (en dispersion à 40% dans le Finsolv TN, benzoate d'alcools en C₁₂-C₁₅) ;
- ceux commercialisés sous la dénomination "DAITOPERSION ZN-30" et "DAITOPERSION ZN-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD ULTRAFINE ZNO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "FUJI ZNO-SMS-10" par la société Fuji Pigment (ZnO enrobé silice et polymethylsilsesquioxane) ;
- ceux commercialisés sous la dénomination "NANOX GEL TN" par la société Elementis (ZnO dispersé à 55% dans du benzoate d'alcools en C₁₂-C₁₅ avec polycondensat d'acide hydroxystéarique).

Les nanopigments d'oxyde de cérium non enrobé est vendu sous la dénomination "COLLOIDAL CERIUM OXIDE" par la société RHONE POULENC.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les nanopigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les nanopigments peuvent être introduits dans les compositions selon l'invention tels quels ou sous forme de pâte pigmentaire, c'est-à-dire en mélange avec un dispersant, comme décrit par exemple dans le document GB-A-2206339.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer /isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Parmi les actifs, on peut citer :
- les agents anti-pollution et/ou agent anti-radicalaire ;
- les agents dépigmentants et/ou des agents pro-pigmentants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants ;
- les agents tenseurs.
- les agents desquamants ;
- les agents hydratants ;
- les agents anti-inflammatoires ;
- les agents agissant sur le métabolisme énergétique des cellules.
- les agents répulsifs contre les insectes
- les antagonistes de substances P ou de CRGP.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile. Elles peuvent se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin et des produits de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés.

### Exemples

On a réalisé les formulations solaires suivantes ; les quantités sont indiquées en pourcentages en poids :

| **Compositions** | **Exemple 1** |
|---|---|
| **PHASE A** | |
| Polydiméthylsiloxane | 0,5 |
| Conservateurs | 1,0 |
| Acide stéarique | 1,5 |
| Mélange monostéarate de glycéryle/stéarate- | 1,0 |
| PEG (100 OE) | |
| Mélange cetylstéarylglucoside/alcool | 2,0 |
| cetylstéarylique | |
| Alcool cétylique | 0,5 |
| Butyl Methoxy Dibenzoylmethane | 2,0 |
| 2-phenylethyl benzoate | 15 |
| (X-TEND 226 de ISP) | |
| Bis-Ethylhexyloxyphenol Methoxyphenyl | 5 |
| Triazine (TINOSORB S par CIBA GEIGY) | |

| **PHASE B** | |
|---|---|
| Eau désionisée | qsp 100 |
| Sequestrant | 0,1 |
| Glycérine | 5,0 |
| Gomme de Xanthane | 0,2 |
| Phosphate de monocétyle | 1,0 |

| **PHASE C** | |
|---|---|
| lsohexadécane | 1,0 |
| Copolymère acide acrylique/méthacrylate de | 0,2 |
| stéaryle | |
| Triéthanolamine | qs |

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotor-stator (appareil de la société Moritz). On incorpore la phase C et on laisse revenir à température ambiante sous agitation modérée. On introduit la triéthanolamine de façon à ajuster le pH à la valeur désirée en fin de fabrication.

## Revendications

1. Procédé de photostabilisation vis-à-vis du rayonnement UV du 4-(tert.-butyl)-4'-méthoxydibenzoylméthane, **caractérisé par le fait qu'**il consiste à associer audit dibenzoylméthane le composé 2-phenylethyl benzoate de formule : 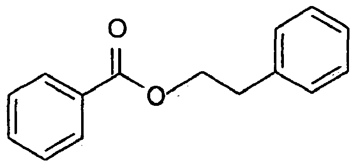 arylalkyl benzoate

2. Composition cosmétique ou dermatologique, **caractérisée par le fait qu'**elle comprend au moins, dans un support cosmétiquement acceptable :
(a) le 4-(tert.-butyl)-4'-méthoxydibenzoylméthane et
(b) le 2-phenylethyl benzoate de formule : 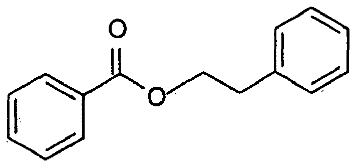
(c) le composé bis-résorcinyl triazine : 2,4-bis {[4-(2-éthyl-hexyloxy)-2-hydroxy]-phényl}-6-(4-méthoxy-phényl)-1,3,5-triazine ; à l'exclusion des compositions suivantes dans lesquelles les quantités des ingrédients sont exprimées en pourcentage en poids par rapport au poids total de la composition :
| **Ingrédients** | **Composition 1** |
|---|---|
| PEG-40 Castor Oil, Sodium Cetearyl Sulfate, Cetearyl Alcohol | 2,50 |
| Alcool stéarylique | 0,50 |
| Alcool cétylique | 2,00 |
| Butylmethoxy Dibenzoylmethane | 4,00 |
| Bis-Ethylhexyloxyphenol Methoxyphenyl Triazin | 0,50 |
| Phenylbenzimidazole Sulfonic Acid | 3,00 |
| Octocrylene | 2,50 |
| Ethylhexyl Salicylate | 5,00 |
| 2-phenylethyl Benzoate | 10,00 |
| Cetearyl Isononanoate | 2,00 |
| Cyclomethicone | 0,50 |
| Dimethicon/Vinyl Dimethicon Crosspolymer | 0,50 |
| Glycerin | 20,00 |
| Gomme de xanthane | 0,30 |
| Vitamine E Acetate | 1,00 |
| Phenoxyethanol | 0,60 |
| EDTA | 0,03 |
| Ethanol | 1,00 |
| Parfum | 0,40 |
| Eau | qsp 100 |
| Agent neutralisant (Soude, Potasse) | qs |
| pH | 5,0-7,5 |
| **Ingrédients** | **Composition 2** |
|---|---|
| Glyceryl Stearate, Ceteareth-12, Ceteareth-20, Cetearyl Alcohol, Cetyl Palmitate | 1,50 |
| Alcool stéarylique | 1,50 |
| Alcool cétylique | 2,00 |
| 2-phenylethyl Benzoate | 10,00 |
| Butylmethoxy Dibenzoylmethane | 4,50 |
| Bis-Ethylhexyloxyphenol Methoxyphenyl Triazin | 0,50 |
| Octocrylen | 2,50 |
| Ethylhexyl Salicylate | 5,00 |
| Diethylhexylnaphthalate (Coropan TQ) | 6,00 |
| C12-C15 Alkyl Benzoate | 5,00 |
| Cetearyl Isononanoate | 2,00 |
| Cyclomethicone | 0,50 |
| Dimethicon/Vinyl Dimethicon Crosspolymer | 0,50 |
| PVP Hexadecen Copolymer | 1,00 |
| Glycerin | 20,00 |
| Gomme de xanthane | 0,30 |
| Vitamine E Acetate | 1,00 |
| Phenoxyethanol | 0,60 |
| EDTA | 0,03 |
| Alcool | 1,00 |
| Parfum | 0,40 |
| Eau | qsp 100 |
| Agent neutralisant (NaOH, KOH) | qs |
| pH | 4,0-7,5 |

3. Composition selon la revendication 2, où le composé dibenzoylméthane est présent à des teneurs allant de 0,01 à 10% en poids et plus préférentiellement de 0,1 à 6% en poids par rapport au poids total de la composition.

4. Composition selon l'une quelconque des revendications 2 à 3, où le dérivé arylalkyl benzoate est présent à des teneurs allant de 0,1 à 40% en poids et plus préférentiellement de 1 à 30 % en poids par rapport au poids total de la composition.

5. Composition selon l'une quelconque des revendications 2 à 4, où le composé bis-résorcinyl triazines est présents à des teneurs allant de 0,1 à 20% en poids et plus préférentiellement de 1 à 10% en poids et plus particulièrement de 2 à 8% en poids par rapport au poids total de la composition.

6. Composition selon l'une quelconque des revendications 2 à 5, **caractérisée par le fait qu'**elle contient en plus d'autres agents photoprotecteurs organiques ou inorganiques actifs dans l'UV-A et/ou l'UV-B hydrosolubles ou liposolubles ou bien insolubles dans les solvants cosmétiques couramment utilisés.

7. Composition selon la revendication 6, où les agents photoprotecteurs organiques complémentaires sont choisis parmi les dérivés cinnamiques, les anthranilates ; les dérivés salicyliques, les dérivés du camphre ; les dérivés de triazine autres que ceux du type bis-résorcinyl triazine ; les dérivés de la benzophénone ; les dérivés de β, β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

8. Composition selon la revendication 7, **caractérisée par le fait que** le ou les filtres UV organiques sont choisis parmi les composés suivants :
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Ethylhexyl triazone,
Diethylhexyl Butamido Triazone,
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

9. Composition selon la revendication 6, **caractérisée par le fait que** les agents photoprotecteurs inorganiques complémentaires sont des pigments ou des nanopigments d'oxydes métalliques, traités ou non.

10. Composition selon la revendication 9, **caractérisée par le fait que** lesdits pigments ou nanopigments sont choisis parmi les oxydes de titane, de zinc, de fer, de zirconium, de cérium et leurs mélanges, traités ou non.

11. Composition selon l'une quelconque des revendications 2 à 10, **caractérisée par le fait qu'**elle comprend en outre au moins un agent de bronzage et/ou de brunissage artificiel de la peau.

12. Composition selon l'une quelconque des revendications 2 à 11, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

## Patentansprüche

1. Verfahren zur Photostabilisierung von 4-(tert.-Butyl)-4'-methoxydibenzoylmethan gegen UV-Strahlung, **dadurch gekennzeichnet, dass** man mit dem Dibenzoylmethan die Benzoesäure-2-phenylethyl-ester-Verbindung der Formel: 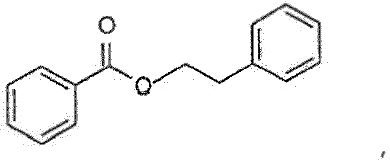 Benzoesäurearylalkylester und die Bisresorcinyltriazin-Verbindung 2,4-Bis{[4-(2-ethylhexyloxy)-2-hydroxy]phenyl}-6-(4-methoxyphenyl)-1,3,5-triazin kombiniert.

2. Kosmetische oder dermatologische Zusammensetzung, **dadurch gekennzeichnet, dass** sie in einem kosmetisch unbedenklichen Träger mindestens Folgendes umfasst:
(a) 4-(tert.-Butyl)-4'-methoxydibenzoylmethan und
(b) Benzoesäure-2-phenylethylester der Formel:
und 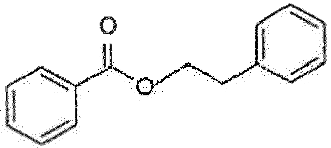
(c) die Bisresorcinyltriazin-Verbindung 2,4-Bis{[4-(2-ethylhexyloxy)-2-hydroxy]phenyl}-6-(4-methoxyphenyl)-1,3,5-triazin;
unter Ausschluss der folgenden Zusammensetzungen, in denen die Mengen der Bestandteile in Gewichtsprozent, bezogen auf das Gesamtgewicht der Zusammensetzung, ausgedrückt sind:
| **Bestandteile** | **Zusammensetzung 1** |
|---|---|
| PEG-40 Castor Oil, Natrium Cetearyl Sulfat, Cetearyl Alkohol | 2,50 |
| Stearyl Alkohol | 0,50 |
| Cetyl Alkohol | 2,00 |
| Butylmethoxydibenzoylmethan | 4,00 |
| Bis-Ethylhexyloxyphenol Methoxyphenyl Triazin | 0,50 |
| Phenylbenzimidazol Sulfonsäure | 3,00 |
| Octocrylen | 2,50 |
| Ethylhexylsalicylat | 5,00 |
| 2-Phenylethyl Benzoat | 10,00 |
| Cetearyl Isononanoat | 2,00 |
| Cyclomethicon | 0,50 |
| Dimethicon/Vinyl Dimethicon Crosspolymer | 0,50 |
| Glycerin | 20,00 |
| Xanthangummi | 0,30 |
| Vitamin E Acetat | 1,00 |
| Phenoxyethanol | 0,60 |
| EDTA | 0,03 |
| Ethanol | 1,00 |
| Parfüm | 0,40 |
| Wasser | ad 100 |
| Neutralisationsmittel (Natriumhydroxid, Kaliumhydroxid) | q.s. |
| pH-Wert | 5,0-7,5 |
| **Bestandteile** | **Zusammensetzung 2** |
|---|---|
| Glyceryl Stearat, Ceteareth-12, Ceteareth-20, Cetearyl Alkohol, Cetyl Palmitat | 1,50 |
| Stearyl Alkohol | 1,50 |
| Cetyl Alkohol | 2,00 |
| 2-Phenylethyl Benzoat | 10,00 |
| Butylmethoxydibenzoylmethan | 4,50 |
| Bis-Ethylhexyloxyphenol Methoxyphenyl Triazin | 0,50 |
| Octocrylen | 2,50 |
| Ethylhexylsalicylat | 5,00 |
| Diethylhexylnaphthalat (Corapan TQ) | 6,00 |
| C₁₂-C₁₅ Alkyl Benzoat | 5,00 |
| Cetearyl Isononanoat | 2,00 |
| Cyclomethicon | 0,50 |
| Dimethicon/Vinyl Dimethicon Crosspolymer | 0,50 |
| PVP Hexadecen Copolymer | 1,00 |
| Glycerin | 20,00 |
| Xanthangummi | 0,30 |
| Vitamin E Acetat | 1,00 |
| Phenoxyethanol | 0,60 |
| EDTA | 0,03 |
| Alkohol | 1,00 |
| Parfüm | 0,40 |
| Wasser | ad 100 |
| Neutralisationsmittel (NaOH, KOH) | q.s. |
| pH-Wert | 4,0-7,5 |

3. Zusammensetzung nach Anspruch 2, wobei die Dibenzoylmethanverbindung in Gehalten im Bereich von 0,01 bis 10 Gew.-% und weiter bevorzugt 0,1 bis 6 Gew.-%, bezogen auf das Gesamtgewicht der Zusammensetzung, vorliegt.

4. Zusammensetzung nach einem der Ansprüche 2 bis 3, wobei das Benzoesäurearylalkylesterderivat in Gehalten im Bereich von 0,1 bis 40 Gew.-% und weiter bevorzugt 1 bis 30 Gew.-%, bezogen auf das Gesamtgewicht der Zusammensetzung, vorliegt.

5. Zusammensetzung nach einem der Ansprüche 2 bis 4, wobei die Bisresorcinyltriazin-Verbindung in Gehalten im Bereich von 0,1 bis 20 Gew.-% und weiter bevorzugt 1 bis 10 Gew.-% und spezieller 2 bis 8 Gew.-%, bezogen auf das Gesamtgewicht der Zusammensetzung, vorliegt.

6. Zusammensetzung nach einem der Ansprüche 2 bis 5, **dadurch gekennzeichnet, dass** sie außerdem andere UV-A- und/oder UV-B-aktive organische oder anorganische Lichtschutzmittel, die wasserlöslich oder fettlöslich oder auch in gemeinhin verwendeten kosmetischen Lösungsmitteln unlöslich sind, enthält.

7. Zusammensetzung nach Anspruch 6, wobei die zusätzlichen organischen Lichtschutzmittel aus Zimtsäurederivaten, Anthranilaten; Salicylsäure-derivaten, Campherderivaten; Triazinderivaten, die von denjenigen des Bisresorcinyltriazin-Typs verschieden sind; Benzophenonderivaten; β,β-Diphenylacrylatderivaten; Benzotriazolderivaten; Benzalmalonatderivaten; Benzimidazolderivaten; Imidazolinen; Bisbenzazolylderivaten; p-Aminobenzoesäurederivaten (PABA-Derivaten); Methylen-bis(hydroxyphenylbenzotriazol)derivaten; Benzoxazolderivaten; Filterpolymeren und Filtersilikonen; von α-Alkylstyrol abgeleiteten Dimeren; 4,4-Diarylbutadienen und Mischungen davon ausgewählt sind.

8. Zusammensetzung nach Anspruch 7, **dadurch gekennzeichnet, dass** das organische UV-Filter bzw. die organischen UV-Filter aus den folgenden Verbindungen ausgewählt ist bzw. sind:
Homosalat,
Ethylhexylsalicylat,
Octocrylen,
Phenylbenzimidazolsulfonsäure,
Benzophenon-3,
Benzophenon-4,
Benzophenon-5,
2-(4-Diethylamino-2-hydroxybenzoyl)benzoesäure-n-hexylester,
4-Methylbenzylidencampher,
Terephthalylidendicamphersulfonsäure,
Dinatriumphenyldibenzimidazoltetrasulfonat,
Methylenbisbenzotriazolyltetramethylbutylphenol,
Ethylhexyltriazon,
Diethylhexylbutamidotriazon,
Drometrizol-Trisiloxan,
Polysilikon-15,
1,1-Dicarboxy-(2,2'-dimethylpropyl)-4,4-diphenyl-butadien,
2,4-Bis-[5-1-(dimethylpropyl)benzoxazol-2-yl-(4-phenyl)imino]-6-(2-ethylhexyl)imino-1,3,5-triazin,
und ihren Mischungen.

9. Zusammensetzung nach Anspruch 6, **dadurch gekennzeichnet, dass** es sich bei den zusätzlichen anorganischen Lichtschutzmitteln um behandelte oder unbehandelte Metalloxidpigmente oder -nanopigmente handelt.

10. Zusammensetzung nach Anspruch 9, **dadurch gekennzeichnet, dass** die Pigmente oder Nanopigmente aus behandelten oder unbehandelten Oxiden von Titan, Zink, Eisen, Zirconium, Cer und Mischungen davon ausgewählt sind.

11. Zusammensetzung nach einem der Ansprüche 2 bis 10, **dadurch gekennzeichnet, dass** sie außerdem mindestens ein Mittel zur künstlichen Bräunung und/oder Braunfärbung der Haut umfasst.

12. Zusammensetzung nach einem der Ansprüche 2 bis 11, **dadurch gekennzeichnet, dass** sie außerdem mindestens einen Hilfsstoff umfasst, der aus Fettsubstanzen, organischen Lösungsmitteln, ionischen oder nichtionischen, hydrophilen oder lipophilen Verdickungsmitteln, zartmachenden Mitteln, Feuchtigkeitsspendern, Trübungsmitteln, Stabilisatoren, Emollientien, Silikonen, Antischaummitteln, Duftstoffen, Konservierungs-mitteln, anionischen, kationischen, nichtionischen, zwitterionischen oder amphoteren Tensiden, Wirkstoffen, Füllstoffen, Polymeren, Treibmitteln, Alkalinisierungsmitteln oder Ansäuerungsmitteln ausgewählt ist.

## Claims

1. Method for photostabilizing 4-(tert-butyl)-4'-methoxydibenzoylmethane with respect to UV radiation, **characterized in that** it consists of combining, with said dibenzoylmethane, the 2-phenylethyl benzoate compound of formula: 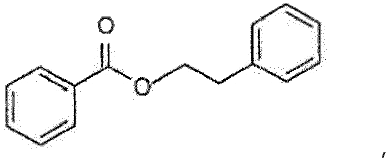 arylalkyl benzoate, and the bis-(resorcinyl)triazine compound: 2,4-bis{[4-(2-ethylhexyloxy)-2-hydroxy]-phenyl}-6-(4-methoxyphenyl)-1,3,5-triazine.

2. Cosmetic or dermatological composition, **characterized in that** it comprises at least, in a cosmetically acceptable support:
(a) 4-(tert-butyl)-4'-methoxydibenzoylmethane and
(b) the 2-phenylethyl benzoate of formula: 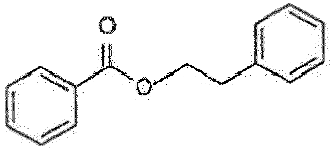 and
(c) the bis(resorcinyl)triazine compound: 2,4-bis{[4-(2-ethylhexyloxy)-2-hydroxy]phenyl}-6-(4-methoxyphenyl)-1,3,5-triazine; excluding the following compositions in which the amounts of the ingredients are expressed as percentage by weight relative to the total weight of the composition:
| **Ingredients** | **Composition 1** |
|---|---|
| PEG-40 castor oil, sodium cetearyl sulfate, cetearyl alcohol | 2.50 |
| Stearyl alcohol | 0.50 |
| Cetyl alcohol | 2.00 |
| Butyl methoxydibenzoylmethane | 4.00 |
| Bis-ethylhexyloxyphenol methoxyphenyl triazine | 0.50 |
| Phenylbenzimidazole sulfonic acid | 3.00 |
| Octocrylene | 2.50 |
| Ethylhexyl salicylate | 5.00 |
| 2-phenylethyl benzoate | 10.00 |
| Cetearyl isononanoate | 2.00 |
| Cyclomethicone | 0.50 |
| Dimethicone/vinyl dimethicone crosspolymer | 0.50 |
| Glycerin | 20.00 |
| Xanthan gum | 0.30 |
| Vitamin E acetate | 1.00 |
| Phenoxyethanol | 0.60 |
| EDTA | 0.03 |
| Ethanol | 1.00 |
| Fragrance | 0.40 |
| Water | q.s. 100 |
| Neutralizing agent (sodium hydroxide, potassium hydroxide) | q.s. |
| pH | 5.0-7.5 |
| **Ingredients** | **Composition 2** |
|---|---|
| Glyceryl stearate, Ceteareth-12, Ceteareth-20, Cetearyl alcohol, Cetyl palmitate | 1.50 |
| Stearyl alcohol | 1.50 |
| Cetyl alcohol | 2.00 |
| 2-phenylethyl benzoate | 10.00 |
| Butyl methoxydibenzoylmethane | 4.50 |
| Bis-ethylhexyloxyphenol methoxyphenyl triazine | 0.50 |
| Octocrylene | 2.50 |
| Ethylhexyl salicylate | 5.00 |
| Diethylhexyl naphthalate (Corapan TQ) | 6.00 |
| C₁₂-C₁₅ alkyl benzoate | 5.00 |
| Cetearyl isononanoate | 2.00 |
| Cyclomethicone | 0.50 |
| Dimethicone/vinyl dimethicone crosspolymer | 0.50 |
| PVP hexadecene copolymer | 1.00 |
| Glycerin | 20.00 |
| Xanthan gum | 0.30 |
| Vitamin E acetate | 1.00 |
| Phenoxyethanol | 0.60 |
| EDTA | 0.03 |
| Alcohol | 1.00 |
| Fragrance | 0.40 |
| Water | q.s. 100 |
| Neutralizing agent (NaOH, KOH) | q.s. |
| pH | 4.0-7.5 |

3. Composition according to Claim 2, in which the dibenzoylmethane compound is present at contents ranging from 0.01 to 10% by weight, and more preferably from 0.1 to 6% by weight relative to the total weight of the composition.

4. Composition according to either one of Claims 2 and 3, in which the arylalkyl benzoate derivative is present at contents ranging from 0.1 to 40% by weight and more preferably from 1 to 30% by weight relative to the total weight of the composition.

5. Composition according to any one of Claims 2 to 4, in which the bis(resorcinyl)triazine compound is present at contents ranging from 0.1 to 20% by weight and more preferably from 1 to 10% by weight and more particularly from 2 to 8% by weight relative to the total weight of the composition.

6. Composition according to any one of Claims 2 to 5, **characterized in that** it also contains other organic or inorganic photoprotective agents, which are active in the UV-A and/or UV-B ranges and are water-soluble or liposoluble or else insoluble in commonly used cosmetic solvents.

7. Composition according to Claim 6, in which the supplementary organic photoprotective agents are chosen from cinnamic derivatives, anthranilates, salicylic derivatives, camphor derivatives, triazine derivatives other than those of the bis(resorcinyl)triazine type, benzophenone derivatives, β,β-diphenylacrylate derivatives, benzotriazole derivatives, benzalmalonate derivatives, benzimidazole derivatives, imidazolines, bis(benzoazolyl) derivatives, p-aminobenzoic acid (PABA) derivatives, methylenebis(hydroxyphenylbenzotriazole) derivatives, benzoxazole derivatives, screening polymers and screening silicones, dimers derived from α-alkylstyrene, 4,4-diarylbutadienes and mixtures thereof.

8. Composition according to Claim 7, **characterized in that** the organic UV screening agent(s) are chosen from the following compounds:
homosalate,
ethylhexyl salicylate,
octocrylene,
phenylbenzimidazole sulfonic acid,
benzophenone-3,
benzophenone-4,
benzophenone-5,
n-hexyl 2-(4-diethylamino-2-hydroxybenzoyl)benzoate,
4-methylbenzylidene camphor,
Terephthalylidene dicamphor sulfonic acid, ,
disodium phenyl dibenzimidazole tetrasulfonate,
methylenebis(benzotriazolyl)tetramethylbutylphenol,
ethylhexyl triazone,
diethylhexyl butamido triazone,
drometrizole trisiloxane,
polysilicone-15,
1,1-dicarboxy(2,2'-dimethylpropyl)-4,4-diphenylbutadiene,
2,4-bis[5-(1-dimethylpropyl)benzoxazol-2-yl-(4-phenyl)imino]-6-(2-ethylhexyl)imino-1,3,5-triazine,
and mixtures thereof.

9. Composition according to Claim 6, **characterized in that** the supplementary inorganic photoprotective agents are treated or untreated metal oxide pigments or nanopigments.

10. Composition according to Claim 9, **characterized in that** said pigments or nanopigments are chosen from treated or untreated oxides of titanium, zinc, iron, zirconium or cerium and mixtures thereof.

11. Composition according to any one of Claims 2 to 10, **characterized in that** it also comprises at least one agent for the artificial tanning and/or bronzing of the skin.

12. Composition according to any one of Claims 2 to 11, **characterized in that** it also comprises at least one adjuvant chosen from fatty substances, organic solvents, ionic or nonionic, hydrophilic or lipophilic thickeners, softeners, humectants, opacifiers, stabilizers, emollients, silicones, antifoaming agents, fragrances, preserving agents, anionic, cationic, nonionic, zwitterionic or amphoteric surfactants, active agents, fillers, polymers, propellants and basifying or acidifying agents.
